# FASCICULE DE BREVET EUROPEEN

(11) **EP 4 076 006 B1**
(45) Date de publication et mention de la délivrance du brevet: **09.12.2026**
(21) Numéro de dépôt: 20848808.0
(22) Date de dépôt: 18.12.2020
(51) Int. Cl.: A23J 1/14, A23K 10/12, A23K 10/30, A23K 20/147, A23K 20/163, A23K 50/00, A23L 11/50, A23L 33/135, C12N 1/205, C12P 13/00, C12P 21/00, A23L 11/30, C12N 1/20, C12P 19/04, C12P 19/12, A23K 20/105

(54) **SOLUBLES DE POIS FERMENTES**
VERGORENE ERBSENLÖSER
FERMENTED PEA SOLUBLES

(30) Priorité: 19.12.2019 FR 1914964; 17.08.2020 FR 2008525
(43) Date de publication de la demande: 26.10.2022
(73) Titulaire: ROQUETTE FRERES, 62136 Lestrem (FR)
(72) Inventeur: HUCHETTE-DEFRETIN, Sophie, 62400 Bethune (FR); MACQUART, Gabriel, 62350 Mont Bernanchon (FR)
(74) Mandataire: Roquette IP International
(86) Numéro de dépôt international: PCT/FR2020/052547
(87) Numéro de publication internationale: WO 2021/123675

(56) Documents cités:
- WO-A1-2013/011216
- FR-A1- 2 897 239
- US-A- 6 159 715
- LABIA IRÈNE IVETTE OUOBA ET AL: "Degradation of polysaccharides and non-digestible oligosaccharides by Bacillus subtilis and Bacillus pumilus isolated from Soumbala, a fermented African locust bean (Parkia biglobosa) food Condiment", EUROPEAN FOOD RESEARCH AND TECHNOLOGY, SPRINGER, BERLIN HEIDELBERG, vol. 224, no. 6, 2 August 2006 (2006-08-02), pages 689 - 694, XP019488853, ISSN: 1438-2385
- MULYOWIDARSO R K ET AL: "CHANGES IN THE CONCENTRATION OF CARBOHYDRATES DURING THE SOAKING OF SOYBEANS FOR TEMPE PRODUCTION", INTERNATIONAL JOURNAL OF FOOD SCIENCE AND TECHNOLOGY, BLACKWELL SCIENTIFIC PUBLICATIONS, OXFORD, GB, vol. 26, no. 6, 1 January 1991 (1991-01-01), pages 595 - 606, XP001015396, ISSN: 0950-5423

## Description

La présente invention concerne un extrait hydrosoluble de pois fermenté dont la composition nutritionnelle est optimisée, en limitant les pertes de matière première et en valorisant au maximum la fraction hydrosoluble. L'invention concerne également son procédé de préparation, ainsi que ses utilisations en industries de la nutrition humaine et animale.

La présente invention concerne également une nouvelle souche de *Bacillus subtilis,* notamment utile pour obtenir un extrait hydrosoluble de pois fermenté.

### Problème technique

Les légumineuses constituent une matière première de choix pour l'industrie agro-alimentaire, notamment pour être consommées après avoir été cuisinées, mais également pour la production de protéines, d'amidon notamment riche en amylose, de fibres et de dérivés de l'amidon tels que les sirops de glucose, la maltodextrine, le dextrose ou l'isoglucose.

Ces produits trouvent des débouchés dans des domaines variés, tels que les secteurs des adhésifs ou du papier, mais surtout dans le domaine alimentaire, où l'intérêt nutritionnel des légumineuses, aussi bien dans l'alimentation humaine qu'animale, n'est plus à démontrer. Parmi celles-ci, les légumineuses à graines, telles que le haricot, le pois et la fève, sont ainsi largement utilisées pour leur apport en énergie et en protéines. Les graines de pois sec sont en effet riches en glucides, formées essentiellement d'amidon et également de saccharose et d'oligosaccharides, en protéines (à teneur élevée en lysine) et en fibres.

En contrepartie de leurs bénéfices nutritionnels, les légumineuses tels que le pois sec présentent une mauvaise digestibilité qui nécessite souvent de les faire tremper en milieu acide et/ou avec présence de bicarbonate de sodium avant de les cuire et de les consommer. Cet inconvénient est principalement attribué à leur teneur significative en alpha-galactosyl oligosaccharides (ou GOS) constitués d'unités D-galactose, D-glucose et D-fructose. En effet, ces oligosaccharides, qui ne sont pas digestibles par les enzymes humaines (incapables de dégrader leurs liaisons alpha1,6-galactosidiques et 1-3 /1-4 fructosidiques), sont transportés intacts jusque dans le côlon où ils fournissent un substrat pour la fermentation de bactéries du microbiote intestinal, provoquant des phénomènes de flatulence. Ce phénomène a notamment été attribué, selon certains auteurs, dans le cas des haricots *(Phaseolus vulgaris),* au motif terminal fructose du raffinose qu'ils contiennent (MYHARA RM et al., Can. Inst. Food Sci. Technol. J., Vol. 21, n° 3, pp. 245-250, 1988).

Ces oligosaccharides sont donc généralement éliminés soit par voie de sélection agronomique de lignées (notamment de soja ou de haricot) à teneur réduite en tels oligosaccharides (BURBANO C. et al., J. Sci. Food Agric., Vol. 79, pp. 1468-1472, 1999), soit par séparation et élimination physique, soit encore par hydrolyse enzymatique (à l'aide d'une alpha-galactosidase) ou fermentaire, effectuée en général préalablement à la consommation de ces légumineuses, mais aussi par administration de compléments alimentaires constitués d'enzymes destinées à hydrolyser ces oligosaccharides en des composés digestibles, avant leur arrivée dans le côlon (US-5,651,967).

Le document US-4,008,334 propose ainsi un procédé pour éliminer les carbohydrates solubles des protéines végétales, issues en particulier du soja, dont la raffinose et le stachyose, par digestion enzymatique à l'aide d'une levure boulangère. Il est intéressant de noter que dans la table 1 de ce document, *Bacillus subtilis* est décrit comme incapable d'hydrolyser le raffinose et le stachyose. De manière analogue, le document US-4,216,235 suggère l'utilisation d'une levure *Saccharomyces uvarum* pour dégrader les oligosaccharides du soja, y compris le mélibiose et le manninotriose. Ces méthodes, bien que parfaitement fonctionnelles, ont pour conséquence l'hydrolyse totale des oligosaccharides, pourtant très utiles en nutrition humaine et animale.

Le document US 2004/0198965 suggère d'utiliser les oligosaccharides, présents notamment dans les graines de soja, pour la synthèse de D-galactose.

Plus précisément pour le pois, le document WO2010/109093 propose l'utilisation d'une invertase afin de défructosyler les oligosaccharides. Les GOS sont ainsi préservés et valorisés par cette solution. Toutefois, le fructose libéré reste libre en solution. Ce sucre est assez décrié nutritionnellement, voire problématique pour certains consommateurs, comme par exemple les consommateurs atteints d'intolérance héréditaire au fructose (IHF). L'IHF est une pathologie autosomique récessive liée au métabolisme du fructose. Elle résulte d'un déficit de l'activité de l'enzyme fructose-1-phosphate aldolase hépatique et conduit à des troubles gastro-intestinaux et à une hypoglycémie postprandiale après ingestion de fructose. Il est donc nécessaire de s'en débarrasser, en ajoutant des étapes de purification coûteuses et longues. De plus, les protéines éliminées en préambule de la réaction de défructosylation sont également à valoriser séparément. Ce procédé est donc complexe, et difficile à commercialiser du fait des différentes fractions générées.

FR 2 897 239 A1 décrit un procédé d'obtention d'une fraction riche en manninotriose et melibiose à partir de graines de soja, comprenant au moins une étape d'extraction de jus de soja et une étape d'obtention d'un perméat à partir dudit jus de soja, caractérisé en ce qu'il comprend au moins une étape d'hydrolyse dudit perméat par voie fermentaire réalisée à l'aide d'au moins une levure boulangère et à l'issue de laquelle ou desquelles ledit ou lesdits extraits actifs sont obtenus.

US 6 159 715 A décrit un procédé de préparation d'une matière oléagineuse modifiée (comprenant au moins 55% de protéines) comportant les étapes suivantes: fourniture d'une matière oléagineuse; et incubation de la matière oléagineuse à une température comprise entre environ 25°C et environ 60°C en présence d'oxygène et de bactéries sporulées (par exemple Bacillus subtilis).

L'invention vise à proposer une autre solution de valorisation de ces fractions hydrosolubles du pois. Précisément, il est apparu au Demandeur que les fractions hydrosolubles de pois peuvent être valorisées en étant fermentées à l'aide d'un protocole très précis par certains microorganismes. L'invention décrite ci-dessous permet donc d'envisager une valorisation des extraits hydrosolubles, en une seule fraction intègre, et à l'aide d'un procédé simple et naturel.

### Description générale de l'invention

L'invention a pour objet une fraction hydrosoluble extraite de légumineuses comportant entre 10% et 30% d'oligosaccharides défructosylés, préférentiellement entre 10% et 25%, préférentiellement entre 15% et 25%, encore plus préférentiellement entre 20% et 22%, et entre 20% et 40% de protéines, préférentiellement entre 25% et 35%, encore plus préférentiellement 30%, les pourcentages étant exprimés en poids sec par rapport au poids total de matière sèche.

L'invention à également pour objet le procédé d'obtention de cette fraction hydrosoluble extraite de légumineuses comportant les étapes suivantes :
1- Obtention d'une fraction hydrosoluble de légumineuses
2- Optionnellement, dessalement de la fraction hydrosoluble
3- Fermentation de la fraction hydrosoluble extraite de légumineuses à l'aide d'un microorganisme du genre *Bacillus,* préférentiellement *Bacillus subtilis*, ladite fermentation étant une fermentation en aérobie avec une pO2 nulle, tout en apportant de l'oxygène en milieu de fermentation directement dans le liquide
4- Optionnellement, élimination du micro-organisme
5- Optionnellement, stabilisation bactériologique de la fraction hydrosoluble ainsi obtenue.

L'invention a enfin pour objet l'utilisation de cette fraction hydrosoluble de légumineuses selon l'invention dans les industries de la nutrition humaine et/ou animale.

Un autre objet de l'invention est aussi une souche de *Bacillus subtilis* telle que déposée le 28 mai 2020 à la CNCM sous le numéro I-5515, ainsi que les différentes utilisations d'une telle souche.

### Description détaillée de l'invention

L'invention a donc pour premier objet une fraction hydrosoluble extraite de légumineuses comportant entre 10% et 30% d'oligosaccharides défructosylés, préférentiellement entre 10% et 25%, préférentiellement entre 15% et 25%, encore plus préférentiellement entre 20% et 22%, et entre 20% et 40% de protéines , préférentiellement entre 25% et 35%, encore plus préférentiellement 30%, les pourcentages étant exprimés en poids sec par rapport au poids total de matière sèche.

Par « fraction hydrosoluble », on entend au sens de la présente invention la fraction aqueuse résiduelle après extraction de l'amidon, des pulpes (aussi appelées fibres internes) et des protéines de type globuline de graines de légumineuses par un procédé de fractionnement dit « humide ». Un tel procédé est par exemple le procédé décrit par la demanderesse dans la demande de brevet EP1400537 incorporée ici par référence. Ce procédé permet d'obtenir les fractions hydrosolubles de pois et les pulpes de pois (cf paragraphes 105 et 106). Il peut être modifié en ajoutant par exemple une étape de trempe, de toastage (chauffage à sec des grains). Cette fraction hydrosoluble résiduelle de légumineuse est principalement composée des protéines solubles à pH acide, majoritairement appartenant au groupe des albumines ainsi que les différents composés hydrosolubles tels que les sucres dont les GOS et les sels. La fraction soluble résiduelle de légumineuse peut subir également un traitement thermique permettant l'élimination de facteurs antinutritionnels tels que les facteurs anti-trypsiques.

Par « légumineuse », on entend au sens de la présente invention la famille de plantes dicotylédones de l'ordre des *Fabales.* C'est l'une des plus importantes familles de plantes à fleurs, la troisième après les *Orchidaceae* et les *Asteraceae* par le nombre d'espèces. Elle compte environ 765 genres regroupant plus de 19 500 espèces. Plusieurs légumineuses sont d'importantes plantes cultivées parmi lesquelles les haricots, les pois, la féverole, le lupin, le haricot, le pois chiche, l'arachide, la lentille cultivée, la luzerne cultivée, différents trèfles, les fèves, le caroubier, la réglisse.

De manière préférée, les légumineuses sont sélectionnées parmi la liste constituée du pois et de la féverole, encore plus préférentiellement du pois.

Le terme « pois » étant ici considéré dans son acception la plus large et incluant en particulier toutes les variétés de « pois lisse » (« smooth pea ») et « de pois ridés » (« wrinkled pea »), et toutes les variétés mutantes de « pois lisse » et de « pois ridé » et ce, quelles que soient les utilisations auxquelles on destine généralement lesdites variétés (alimentation humaine, nutrition animale et/ou autres utilisations). Le terme « pois » dans la présente demande inclut les variétés de pois appartenant au genre *Pisum* et plus particulièrement aux espèces *sativum* et *aestivum.* Lesdites variétés mutantes sont notamment celles dénommées « mutants r », « mutants rb », « mutants rug 3 », « mutants rug 4 », « mutants rug 5 » et « mutants lam » tels que décrits dans l'article de C-L HEYDLEY et al. intitulé « Developing novel pea starches » Proceedings of the Symposium of the Industrial Biochemistry and Biotechnology Group of the Biochemical Society, 1996, pp. 77-87.

Le terme « féverole » s'entend ici par le groupe des plantes annuelles de l'espèce *Vicia faba,* appartenant au groupe des légumineuses de la famille des Fabaceae, sous-famille des Faboideae, tribu des Fabeae. On distingue les variétés Minor et Major. Dans la présente invention, les variétés sauvages et celles obtenues par génie génétique ou sélection variétales sont toutes d'excellentes sources.

Par « oligosaccharides », on entend au sens de la présente invention les oligomères formés d'un nombre n d'oses (monosaccharides) par liaison glycosidique alpha ou beta. Par convention le nombre n varie de 3 à 10. Là, ils sont placés entre les oses simples (n=1) et les polyosides (polysaccharides) (n>10). Cependant cette limite de 10 unités n'est pas totalement figée et les polyosides de degré de polymérisation de 11 à 25 leur sont souvent assimilés. Les oligosides comprenant 2 oses sont les diholosides (saccharose), 3 oses les triholosides (raffinose, mélézitose) et 4 oses les tétraholosides (stachyose). Les oligosides peuvent être linéaires (stachyose), ramifiés ou bien cycliques (cyclodextrine).

De manière préférée, la fraction hydrosoluble selon l'invention comporte des oligosaccharides défructosylés sélectionnés dans la liste contenant le mélibiose, le manninotriose et le verbascotétraose.

Par « mélibiose », on entend au sens de la présente invention le diholoside constitué d'une unité de galactose lié à une unité de glucose par une liaison osidique α(1→6).

Par « manninotriose », on entend au sens de la présente invention le triholoside constitué de l'enchainement d'une unité de galactose lié par une liaison osidique α(1→6) à une autre unité de galactose, elle-même liée à une unité de glucose par une autre liaison α(1→6).

Par « verbascotétraose », aussi appelé « manninotétraose », on entend au sens de la présente invention le tétraholoside constitué de l'enchainement de trois unités de galactose liés par des liaisons osidiques α(1→6), la troisième unité de galactose étant elle-même liée à une unité de glucose par une autre liaison α(1 →6).

Il est remarquable selon l'invention que les oligosaccharides traditionnellement présents dans la fraction hydrosoluble du pois (raffinose, stachyose et verbascose) soient complétement défructosylés, en enrichissant en oligosaccharides défructosylés (mélibiose, manninotriose et verbascotétraose). De plus, la teneur en fructose libre est réduite par rapport à la teneur initiale, malgré la défructosylation. Sans être lié par une quelconque théorie, la souche décrite dans les paragraphes ci-dessous, qui réalise la défructosylation, doit re-consommer celui-ci. L'avantage par rapport à l'art antérieur, en particulier WO2010/109093, est qu'en une seule étape les GOS sont défructosylés et le fructose est éliminé. L'industrie alimentaire a donc à sa disposition une fraction hydrosoluble dont les GOS sont défructosylés et avec une teneur en fructose réduite.

Toute méthode bien connue de l'homme du métier afin de quantifier ces oligosaccharides défructosylés est convenable aux fins de la présente invention. Les méthodes chromatographiques seront privilégiées. De manière préférée, l'homme du métier utilisera la méthode de dosage par ampérométrie HPAEC-PAD et en particulier avec les matériels suivants :
- La pré-colonne Dionex Carbopac PA1 4*50mm - Réf. 43096
- La colonne Dionex Carbopac PA1 4*250mm - Réf. 35391
- Le détecteur est de type PAD, précisément cellule en or

Les éluants sont :
- Solvant A / NaOH 0.1M : Mettre 4 litres d'eau en agitation sous Hélium (débit : 100ml/mn) pendant 15 mn. Ajouter 20 ml de NaOH à 50%. Remettre en agitation sous hélium à 40 ml/mn.
- Solvant B / NaOH 0.1M+0.5M acetate de sodium Peser 82g d'acétate de Na directement dans le bidon. Ajouter 2l d'H2O. Mettre en agitation et sous hélium (débit : 100ml/mn) pendant 15 mn puis ajouter 10 ml de NaOH 50%, remettre en agitation et sous hélium. Le débit de l'hélium peut être baissé à 40 ml/mn.

On utilise des standards pour calibrer l'HPLC et en particulier :

| ***Rèactifs*** | ***Rèfèrence*** |
|---|---|
| Melibiose | Fluka réf 63630 |
| Raffinose | Sigma ref R-0514 |
| Stachyose | Sima ref S-4001 |
| Verbascose | Fluka ref 56217 |

On utilise également un étalon interne : le Panose réf SIGMA P-2407 60mg à 100ml d'eau.

Le volume injecté est de 5µl à une température de 15°C. Le temps d'analyse est de 90 min avec une température de colonne de 30°C et une sensibilité de l'injecteur de 300nC ou 5µA

Les conditions d'élution chromatographiques sont les suivantes :

| Temps (min) | Débit (ml/mn) | **Solvant A** | **Solvant B** |
|---|---|---|---|
| 0 | 0.5 | 98 | 2 |
| 60 | 0.5 | 95 | 5 |
| 65 | 0.5 | 70 | 30 |
| 65.05 | 0.5 | 0 | 100 |
| 75 | 0.5 | 0 | 100 |
| 75.05 | 0.5 | 98 | 2 |
| 90 | 0.5 | 98 | 2 |

Le programme d'oxydation du détecteur PAD est le suivant :

| Temps (min) | Potentiel | Intégration |
|---|---|---|
| 0 | +0.05 | |
| 0.20 | +0.05 | Début |
| 0.40 | +0.05 | Fin |
| 0.41 | +0.75 | |
| 0.60 | +0.75 | |
| 0.61 | -0.15 | |
| 1.0 | -0.15 | |

La calibration est réalisée en préparant 2 courbes suivant le tableau ci-dessous :

| Quantité en mg | Mélibiose QSP 50ml | Raffinose QSP 25ml | Stachyose QSP 25ml | Verbascose QSP 25ml |
|---|---|---|---|---|
| T1 | 10 | 5 | 5 | 5 |
| T2 | 25 | 10 | 10 | 10 |
| T3 | 50 | 15 | 15 | 15 |
| T4 | 75 | 25 | 25 | 25 |
| T5 | 100 | | | |

Prélever 1 ml de témoin (des 2 courbes) + 1 ml Etalon interne, qsp 20ml d'eau.

Peser la quantité en mg d'échantillon, ajouter 1 ml d'étalon interne et ajuster à 20 ml d'eau.

Filtrer sur GxF/GHP 0.45 µm ref 4559T.

De manière avantageuse, la fraction hydrosoluble selon l'invention contient moins de 2 %, de manière préférée entre 0,25% et 1%, encore plus préférentiellement entre 0,5% et 0,75% en poids de fructose, les pourcentages étant exprimés en poids par rapport au poids total de matière sèche.

Toute méthode bien connue de l'homme du métier afin de quantifier le fructose est convenable aux fins de la présente invention. Les méthodes enzymatiques seront privilégiées. Le fructose peut ainsi être dosé par méthode chromatographique HPLC. De manière préférée, l'homme du métier utilisera la méthode suivante :

Les teneurs en glucose dosées par cette méthode, doivent être inférieures à 1 g/L, de même pour les teneurs en fructose, les quantités en glucose + fructose doivent être inférieures à 1 g/L. Dans le cas contraire, une dilution préalable sera effectuée et prise en compte dans le calcul final

Le principe est le suivant :
L'hexokinase (HK) catalyse la phosphorylation du glucose et du fructose par l'adénosine-5-triphosphate (ATP) à pH 7,6.

Dans une réaction catalysée par la glucose-6-phosphate-déshydrogénase(G6P-DH), le glucose-6-phosphate formé (G6P) est oxydé spécifiquement en présence du nicotinamide-adénine-dinucléotide-phosphate (NADP) en gluconate-6-phosphate, avec formation de nicotinamide-adénine-dinucléotide-phosphate réduit (NADPH).

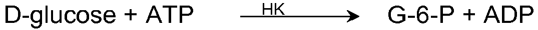

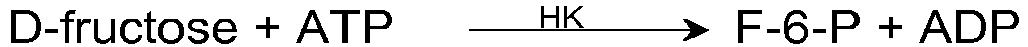

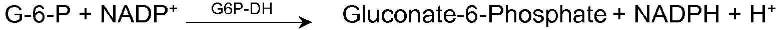

La quantité de NADPH formée au cours de la réaction est proportionnelle à la quantité de glucose.

A la fin de la réaction, le fructose-6-P est transformé en glucose-6-P par la phospho-glucose isomérase.

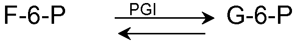

Le G-6-P formé réagit à son tour avec le NADP en formant du gluconate-6-phosphate et du NADPH. La quantité de NADPH formée est mesurée à nouveau. Elle est proportionnelle à la quantité de fructose.

Les réactifs sont les suivants :
- Solution standard de glucose et de fructose à 1g/L : Préparer un standard unique contenant les deux sucres à une concentration de 1g/L.
- Tampon triéthanolamine pH 7,6 : Dans un bécher de 500mL, peser 88,45g de triéthanolamine chlorhydrique, 1185mg de NADP (Roche ref 240 354), 2960mg de ATP (Roche ref 127 523) et 1250mg de MgSO4, 7H2O. Ajuster le pH à 7,6 avec NaOH 4N. Ajuster à 500mL avec de l'eau.
- Hexokinase (HK) : Référence Roche 127 825. A utiliser telle quelle.
- Phosphoglucose-isomérase (PGI) Référence Roche 128 139. A utiliser telle quelle.

Le modé opératoire est le suivant :
- La réaction se fait directement dans les cuves de spectrophotomètre.
- Dans chaque cuve, introduire : 1mL de solution tampon, 1,9mL d'eau et100µL d'échantillon ou 25µL de solution standard (+75µL eau)
- Mélanger. Attendre 5 minutes et lire l'absorbance à 340nm (A₀).
- Ajouter 20µL d'hexokinase .
- Mélanger. Attendre la fin de la réaction (environ 10 minutes) puis lire l'absorbance à 340nm (A₁).
- Ajouter 20µL de phospho-glucose isomérase (3-4).
- Mélanger. Attendre la fin de la réaction (environ 10 minutes) puis lire l'absorbance à 340nm (A₂).
- Si la teneur en glucose de la solution à doser est très faible, augmenter la prise d'essai en remplaçant l'eau par l'échantillon.
- De même pour le fructose, mais il faut tenir compte de la concentration en glucose de la solution à analyser.

La concentration en glucose en g/L de la solution à doser se calcule suivant la formule : $C = \frac{\left⌊{\left({A}_{1}-{A}_{0}\right)}_{ech}-{\left({A}_{1}-{A}_{0}\right)}_{blanc}\right⌋ \times 0 , 5441}{6 , 3 \times v}$ avec v = volume de la prise d'essai échantillon en mL

La concentration en fructose en g/L de la solution à doser se calcule suivant la formule : $C = \frac{\left⌊{\left({A}_{2}-{A}_{1}\right)}_{ech}-{\left({A}_{2}-{A}_{1}\right)}_{blanc}\right⌋ \times 0 , 5477}{6 , 3 \times v}$ avec v = volume de la prise d'essai échantillon en mL

Pour effectuer le dosage du fructose, il faut impérativement effectuer auparavant le dosage de glucose. La différence d'absorbance pour le glucose ne doit pas excéder 1.

Lorsque le ratio glucose/fructose dans un échantillon dépasse 80/20, il est nécessaire, avant de faire le dosage de fructose, de détruire le glucose présent par action de la glucose oxydase (GOD) et de la catalase en présence d'oxygène (voir protocole ci-dessous).

De manière avantageuse, la fraction hydrosoluble selon l'invention contient moins de 2 %, de manière préférée entre 0,25% et 1%, encore plus préférentiellement entre 0,5% et 0,75% en poids de lactate, les pourcentages étant exprimés en poids par rapport au poids total de matière sèche.

L'acide lactique (ou lactate) est un acide carboxylique bien connu constitué d'un squelette carboné de 3 et possédant une fonction hydroxyle sur son carbone central. Il est souvent produit lors de fermentation de microorganismes lorsque l'oxygène est en défaut (p.e. en anaérobie) et que le métabolisme de la souche fermentée passe dans un mode dit fermentaire. Le mode de production de la fraction hydrosoluble selon l'invention permet la limitation de la production de cet acide, indésirable dans plusieurs formulations alimentaires s'il est en teneur trop importante.

Toute méthode bien connue de l'homme du métier afin de quantifier l'acide lactique est convenable aux fins de la présente invention. Les méthodes chromatographiques seront privilégiées. De manière préférée, l'homme du métier utilisera la méthode suivante :
La détermination de la quantité d'acide lactique est réalisé par HPLC en mode isocratique avec :
- pré-colonne AG11-HC - Dionex - Réf. 52962
- colonne AS11-HC - Dionex - Réf. 52960
- détecteur : conductimêtre

Les éluants sont :
- A : Eau purifiée milli-Q (sous Hélium)
- B : Cartouche d'hydroxyde de potassium Dionex - Réf. 58900

Un standard commercial d'acide lactique (DL-Acide Lactique (sel de sodium) Fluka - Réf. 71720) et un étalon interne (Acide Trifluoroacétique (sel de sodium) (solution à 400mg/l) Sigma Réf. T-0757) sont utilisés pour étalonner l'HPLC.

La calibration s'effectue avec 5 points, en pesant entre 5 et 120mg d'acide lactique témoin QSP 500ml d'eau. Prélever 0.5 ml de chaque solution, ajouter 0.5 ml d'étalon interne et ajuster à 20 ml avec de la soude 1mM. Tracer la courbe de calibration par hauteur

Les conditions d'élutions chromatographiques sont les suivantes (la molarité de l'éluant B est réalisée par dilution avec l'éluant A) :

| Temps (min) | Débit (ml/mn) | **Molarité B (mmol/l)** |
|---|---|---|
| 0 | 1.5 | **1.5** |
| 15 | 1.5 | **1.5** |
| 28 | 1.5 | **12** |
| 48 | 1.5 | **28** |
| 60 | 1.5 | **40** |
| 60.1 | 1.5 | **60** |
| 67 | 1.5 | **60** |
| 67.1 | 1.5 | **1.5** |

- Volume injecté : 25µl,
- T° injecteur : 10°C
- Temps d'analyse : 77 min
- ASRS : 300 mA
- T° colonne : 36°C

De manière encore plus préférée, la fraction hydrosoluble selon l'invention comporte entre 20% et 40% de protéines caractérisées comme des albumines, préférentiellement entre 25% et 35%, encore plus préférentiellement 30%, les pourcentages étant exprimés en poids par rapport au poids total de matière sèche.

Par « albumine », on entend au sens de la présente invention les protéines solubles dans l'eau pure. Les albumines de pois, présentes dans les protéines de pois à hauteur d'environ 20%, se subdivisent principalement en deux familles baptisées PA1 et PA2.

Les albumines présentes dans la fraction hydrosoluble selon l'invention possèdent un profil en aminoacides remarquablement conservé, permettant d'offrir une source d'acides aminés très intéressante. Comme il sera exemplifié plus bas, c'est le procédé de l'invention, en particulier la gestion de l'oxygénation, qui permet de garantir à la fois une défructosylation, tout en conservant ce profil quasi identique, à l'exception de l'arginine qui va être convertie en agmatine.

Toute méthode bien connue de l'homme du métier afin de quantifier la quantité de protéine est convenable aux fins de la présente invention. De manière préférée, l'homme du métier utilisera la méthode dite « N6,25 » suivante :
- Dosage de l'azote total en utilisant la méthode de Kjeldahl ou de Dumas, préférentiellement Dumas. L'expression de cet azote étant en gramme d'azote par poids total de l'échantillon
- Multiplication de la teneur en azote précédemment dosée par le coefficient 6,25

De manière préférée, la fraction soluble selon l'invention possède des protéines, préférentiellement des albumines, dont le degré d'hydrolyse, ou DH, est inférieur à 20, préférentiellement inférieur à 18, encore plus préférentiellement inférieur à 15

Par « degré d'hydrolyse » on entend dans la présente invention le rapport en pourcentage entre la quantité de fonctions amines (ou carboxyliques) des acides aminés libres sur la quantité totale, incluant les fonctions libres et celles engagées dans une liaison peptidique (liaison chimique caractéristique des protéines résultant de l'association d'une fonction carboxylique d'un premier acide aminé et d'une fonction amine d'un second). Pour une composition protéique constituée par l'enchainement de tous ses acides aminés et donc présentant seulement une fonction amine et une fonction carboxylique libres, ce degré d'hydrolyse sera de 0%. A l'inverse, pour une composition de protéines dont les mêmes acides aminés seront tous dits « libres » c'est-à-dire dont leurs deux fonctions amines et carboxyliques ne sont pas impliquées dans des liaisons peptidiques, ce degré d'hydrolyse sera de 100%.

Il existe plusieurs méthodes afin de quantifier le degré d'hydrolyse. Elles consistent toutes majoritairement par le dosage colorimétrique des fonctions amines (ou carboxyliques) libres, puis la réalisation d'une hydrolyse visant à détruire l'intégralité des liaisons peptidiques et enfin d'un dosage colorimétrique des fonctions amines (ou carboxyliques) totales. Le pourcentage calculé entre les amines (ou carboxyliques) libres par rapports aux totales donne le degré d'hydrolyse. On pourra utiliser toute méthode bien connue telles que la méthode dite TNBS ou la méthode OPA. Dans la présente invention, on préfèrera la méthode OPA dont un mode opératoire de mesure est décrit ci-dessous :
On détermine tout d'abord la teneur en azote aminé (NH₂ libre) sur l'échantillon de protéines selon l'invention avec le kit MEGAZYME (référence K-PANOPA). On détermine également la teneur en azote protéique (azote total) de l'échantillon. On peut alors calculer le degré d'hydrolyse.

### Détermination de la teneur en azote aminé :

Les groupes « azote aminé » des acides aminés libres de l'échantillon réagissent avec le N-acétyl-L-cystéine et l'OPhthaldialdéhyde (OPA) pour former des dérivés d'isoindole.

La quantité de dérivé d'isoindole formée au cours de cette réaction est stœchiométrique avec la quantité d'azote aminé libre. C'est le dérivé d'isoindole qui est mesuré par l'augmentation de l'absorbance à 340 nm.

Dans un bécher de 100 mL, on introduit une prise d'essai P*, exactement pesée, de l'échantillon à analyser. Cette prise d'essai sera de 0,5 à 5,0 g en fonction de la teneur en azote aminé de l'échantillon. On ajoute environ 50 mL d'eau distillée, on homogénéise et on transvase dans une fiole jaugée de 100 mL. On ajoute 5 mL de dodécyle sulfate de sodium (SDS) à 20% et on complète avec de l'eau distillée pour atteindre un volume de 100 mL. On agite pendant 15 minutes avec un agitateur magnétique à 1000 rpm. On prépare une solution n°1 en dissolvant un comprimé du flacon 1 du kit Megazyme dans 3 mL d'eau distillée et on agite jusqu'à dissolution complète. Il faut prévoir un comprimé par essai. La solution n°1 est préparée extemporanément.

On prépare un blanc, un standard et un échantillon directement dans les cuves du spectrophotomètre dans les conditions suivantes :
- blanc : introduire 3,00 ml de la solution n°1 et 50 µl d'eau distillée
- standard : introduire 3,00 ml de la solution n°1 et 50 µl du flacon 3 du kit Megazyme
- échantillon : introduire 3,00 ml de la solution n°1 et 50 µl de la préparation de l'échantillon.

On mélange le contenu de chaque cuve et on lit la mesure d'absorbance (A1) des solutions après 2 mn environ au spectrophotomètre à 340 nm (spectrophotomètre équipé de cuves de 1,0 cm de trajet optique, pouvant mesurer à une longueur d'onde de 340 nm, et vérifié selon le mode opératoire décrit dans le manuel technique du constructeur qui s'y rapporte).

On amorce ensuite les réactions immédiatement en ajoutant 100 µl de la solution n°2 qui correspond à la solution d'OPA du flacon 2 du kit Megazyme dans chaque cuve de spectrophotomètre.

On mélange le contenu de chaque cuve et on les place environ 20 minutes dans l'obscurité.

On lit ensuite la mesure d'absorbance A2 du blanc, du standard et de l'échantillon au spectrophotomètre à 340 nm.

La teneur en azote aminé libre, exprimée en pourcentage en poids par rapport au poids du produit, est donnée par la formule suivante : $% azote aminé = \frac{\left(ΔAech-ΔAblc\right) \times 3 , 15 \times 14 , 01 \times V \times 100}{6803 \times 0 , 05 \times m \times 1000}$ $% azote amin é = \frac{\left(ΔAech-ΔAblc\right) \times 12 , 974 \times V}{m \times 1000}$ où : $ΔAech = Aech 2 - Aech 1$ $ΔAblc = Ablc 2 - Ablc 1$
Aech2 = absorbance de l'échantillon après ajout de la solution n°2
Aech1 = absorbance de l'échantillon après ajout de la solution n°1
Ablc2 = absorbance du blanc après ajout de la solution n°2
Ablc1 = absorbance du blanc après ajout de la solution n°1
V = volume de la fiole
m = masse de la prise d'essai en g
6803 = coefficient d'extinction du dérivé d'isoindole à 340 nm (en L.mol⁻¹.cm⁻¹).
14,01 = masse molaire de l'azote (en g.mol⁻¹)
3,15 = volume final dans la cuve (en mL)
0,05 = prise d'essai dans la cuve (en mL)

### Détermination de la teneur en azote protéique :

La teneur d'azote protéique est déterminée selon la méthode de DUMAS selon la norme ISO 16634 - 2016. Elle est exprimée en pourcentage en poids par rapport au poids du produit.

### Calcul du degré d'hydrolyse

Le degré d'hydrolyse (DH) est calculé avec la formule suivante : $DH = \frac{% azote amin é}{% azote prot é ique} \times 100$

De manière encore plus préférée, la fraction hydrosoluble selon l'invention comporte également de l'agmatine dans une concentration comprise entre 10 ppm et 100 ppm exprimé en poids sec d'agmatine sur poids sec de produit final, préférentiellement entre10 ppm et 40 ppm sur sec, préférentiellement entre 15 ppm et 35 ppm, encore plus préférentiellement entre 20 ppm et 30 ppm.

Par « agmatine », on entend au sens de la présente invention l'amine biogène obtenue à partir d'arginine par une réaction chimique appelée décarboxylation. Elle est présente dans la plupart des tissus de notre organisme, les végétaux, la viande et le poisson. Ce sous-produit métabolique de l'arginine est stocké dans les cellules du cerveau et de la moelle épinière. L'agmatine favorise la décharge de monoxyde d'azote, une molécule qui intervient dans le relâchement des muscles lisses. Elle permet donc de mieux gérer le stress. Toute méthode connue de l'homme du métier convient pour réaliser ce dosage. On utilisera particulièrement la méthode décrite dans « Improved Method for HPLC Analysis of Polyamines, Agmatine and Aromatic Monoamines in Plant Tissue (Robert D. Slocum & al., Plant Physiol. 1989 Feb; 89(2): 512-517.)

L'invention a également pour objet le procédé d'obtention de cette fraction hydrosoluble extraite de légumineuses comportant les étapes suivantes :
1- Obtention d'une fraction hydrosoluble de légumineuses,
2- Optionnellement, dessalement de la fraction hydrosoluble,
3- Fermentation de la fraction hydrosoluble extraite de légumineuses à l'aide d'un microorganisme du genre *Bacillus,* préférentiellement Bacillus subtilis, ladite fermentation étant une fermentation en aérobie avec une pO2 nulle, tout en apportant de l'oxygène en milieu de fermentation directement dans le liquide,
4- Optionnellement, élimination du micro-organisme,
5- Optionnellement, stabilisation bactériologique de la fraction hydrosoluble ainsi obtenue.

La première étape du procédé selon l'invention consiste donc en l'obtention d'une fraction hydrosoluble de légumineuses.

De manière préférée, cette première étape se divise en deux sous-étapes :
i) Mise en œuvre de graines de légumineuses, avec un pré-traitement optionnel ;
ii) Séparation par voie humide des constituants des graines de légumineuses en 4 fractions : une fraction amidon, une fraction pulpes, une fraction protéines de type globulines et une fraction hydrosoluble soluble résiduelle ;

La première étape i) de mise en œuvre des graines de pois consiste en la préparation pour les étapes suivantes. Les graines peuvent d'abord subir des étapes de nettoyage, de tamisage (séparation des graines des cailloux par exemple.). Ensuite, les fibres externes sont séparées des graines proprement dites (cette étape est également connue sous le terme anglosaxon de dehulling). Enfin, les cotylédons obtenus peuvent subir des étapes de trempage, de blanchiment, de toastage. De manière préférée, si un blanchiment est effectué, le barème sera de 3 min à 80°C.

La seconde étape ii) est décrite précisément dans la demande de brevet EP1400537 qui est incorporée ici par référence. La graine de pois est réduite en farine par broyage et mise en suspension dans de l'eau. Ces deux étapes peuvent être successives dans un procédé dit « broyage à sec » (broyage puis mise en suspension) ou simultanées dans un procédé dit « broyage humide ». L'amidon et les pulpes (appelées aussi fibres internes) sont respectivement éliminés par utilisation d'hydrocyclones et de décanteuses horizontales. Après élimination de ces deux composés insolubles, le surnageant liquide obtenu voit son pH acidifié entre 4 et 6, préférentiellement entre 4,5 et 5, afin de faire précipiter les protéines dites « globulines » (représentant environ 80% des protéines totales), un chauffage entre 50°C et 80°C, préférentiellement entre 60°C et 70°C peut être appliqué consécutivement afin de faire coaguler les globulines avec un rendement maximal. Le coagulat ainsi obtenu est envoyé dans des centrifugeuses afin de séparer les globulines sous forme d'un floc solide d'une part et la fraction hydrosoluble résiduelle sous forme liquide d'autre part. Tout autre procédé d'extraction humide aboutissant en la génération de ces 4 fractions : une fraction amidon, une fraction pulpes, une fraction protéines de type globulines et une fraction hydrosoluble résiduelle peut être également mis en œuvre afin de générer une fraction hydrosoluble. Il est également possible d'obtenir un concentrât par voie sèche (turbo-séparation ou air-classification) puis de continuer l'extraction des différentes fractions par voie humide.

De manière optionnelle, la fraction hydrosoluble de légumineuse peut également subir plusieurs étapes de filtrations membranaires afin de réduire, voire de séparer la fraction protéique, majoritairement constituée d'albumines. La fraction hydrosoluble ainsi préparée sera réduite voire privée de ses albumines. Deux fractions hydrosolubles sont ainsi générées : une fraction albumine et une fraction sucre. Cette séparation des albumines de la fraction hydrosoluble est bien connue par exemple de l'article « Pilot scale recovery of proteins from a pea whey discharge by ultrafiltration » (Gao & al. 2000) ou encore de la demande de brevet WO2014/118449. Pour ce faire, on privilégiera l'utilisation d'une ultrafiltration dont le seuil de coupure est adapté pour séparer protéines et sucres. La fraction sucre servira de matériau brut pour les étapes suivantes. Avec ces deux fractions distinctes (albumines et sucres fermentés selon l'invention), le ratio albumines/sucres pourra être plus finement standardisé en réalisant un mélange des deux fractions. Les quantités résiduelles pourront être valorisées séparément. Selon un mode de réalisation, le procédé d'obtention de la fraction hydrosoluble extraite de légumineuses selon l'invention comporte ainsi les étapes suivantes :
1- Obtention d'une fraction hydrosoluble de légumineuses,
   1bis- Au moins une filtration membranaire de la fraction hydrosoluble,
2- Optionnellement, dessalement de la fraction hydrosoluble,
3- Fermentation de la fraction hydrosoluble extraite de légumineuses à l'aide d'un microorganisme du genre *Bacillus,* préférentiellement Bacillus subtilis, ladite fermentation étant une fermentation en aérobie avec une pO2 nulle, tout en apportant de l'oxygène en milieu de fermentation directement dans le liquide,
4- Optionnellement, élimination du micro-organisme,
5- Optionnellement, stabilisation bactériologique de la fraction hydrosoluble ainsi obtenue.

La seconde étape, optionnelle, selon l'invention consiste en un dessalement de la fraction hydrosoluble de légumineuse. Pour ce faire, toute technique bien connue de l'homme du métier peut être utilisée telle que par exemple la déminéralisation ou la précipitation. De manière préférée, on utilisera une séparation membranaire telle que l'ultrafiltration, la nanofiltration ou l'osmose inverse. Le but est ici de séparer les sels dans le perméat et le reste de la fraction hydrosoluble dans le rétentat. De manière préférée, on utilisera une nanofiltration avec un seuil de coupure d'environ 500 Da, plus précisément compris entre 1 KDa et 250 Da. Cette étape optionnelle est recommandée afin de se débarrasser de la quantité trop importante de sels, en particulier le potassium. La fraction hydrosoluble est ainsi purifiée de son excès de sels (environ 80% en moyenne) qui est concentré dans le perméat. Le rétentat sert ensuite de matière première pour les étapes suivantes.

La troisième étape du procédé selon l'invention consiste donc en la fermentation de la fraction hydrosoluble extraite de légumineuses à l'aide d'un microorganisme du genre *Bacillus,* préférentiellement *Bacillus subtilis,* encore plus préférentiellement *Bacillus subtilis Natto.*

Par « fermentation », on entend selon l'invention les processus métaboliques convertissant généralement des glucides en acides, en gaz ou en alcools pour en extraire une partie de l'énergie chimique tout en ré-oxydant les coenzymes réduites par ces réactions. Il s'agit d'une voie métabolique d'oxydoréduction dans laquelle l'accepteur ultime d'électrons est souvent confondu avec le produit final des réactions. Elle se caractérise par une dégradation partielle de la substance fermentescible et ne permet qu'une production d'énergie limitée. Elle a lieu chez des levures et des bactéries, ainsi que dans les cellules musculaires manquant d'oxygène, c'est-à-dire en conditions anaérobies.

Dans la présente invention, la fermentation est réalisée en milieu liquide, fermentation dite « submergée ».

Comme il sera exemplifié plus bas, la fermentation est réalisée avec une pO2 nulle, tout en apportant de l'oxygène au milieu de fermentation directement dans le liquide.

Par « pO2 », on entend dans la présente invention la teneur en oxygène dissous mesurable à l'aide de sonde adaptée classiquement utilisée en fermentation industrielle. Cette sonde mesure ainsi en temps réel la concentration exacte d'oxygène dissous dans le milieu de fermentation. A noter que la pO2 peut être nulle tout en envoyant en parallèle de l'air ou de l'oxygène dans le fermenteur. L'oxygène introduit est alors immédiatement métabolisé par le microorganisme du genre *Bacillus.*

De manière préférée, l'apport en oxygène se fera par introduction d'un débit d'air compris entre 0,03 VVM et 0,5 VVM, préférentiellement entre 0,1 VVM et 0,4 VVM, encore plus préférentiellement entre 0,2 VVM et 0,3 VVM, 0,25 VVM étant la valeur ciblée préférentiellement.

Par « VVM » on entend dans la présente invention la quantification d'un débit de gaz, préférentiellement d'air ou d'oxygène pur, introduit dans un fermenteur. 1 VVM signifie 1 Volume de gaz par Volume de fermenteur par Minute. Plus précisément, pour un fermenteur d'un m³, 1 VVM signifie 1 m³ de gaz par minute.

De manière préférée, le pH de la fermentation sera rectifié voire régulé entre 5,5 et 6,5 ; préférentiellement 6. En effet, comme démontré dans la partie exemple un pH de fermentation supérieur à 6,5 aura pour conséquence la surproduction d'acide lactique.

De manière préférée, la température de fermentation est comprise entre 30°c et 40°C, préférentiellement entre 32° et 37°C, encore plus préférentiellement 37°C.

De manière encore plus préférée, la fermentation de la fraction hydrosoluble extraite de légumineuses est réalisée avec un milieu de fermentation composé uniquement de ladite fraction hydrosoluble. En effet, il peut être envisageable d'ajouter différents substrats carbonés (p.e. glucose, fructose, amidon) et aminés (pe. Ammoniaque, extrait de levure, sulfate d'ammonium, caséine, lactosérum, protéine de soja) au milieu de fermentation. Dans ce cas, les souches réalisant la fermentation consommeront de manière concurrentielle voire préférentielle, les différents substrats ajoutés. La défructosylation se déroulera de manière moins efficace, moins complète voire ne sera pas réalisée. De même, les protéines pourront être hydrolysées. L'ajout de composés chimiques (comme le sulfate d'ammonium) ou de composé allergènes (comme le soja ou la caséine) devra être indiqué aux consommateurs finaux ce qui pourra avoir des conséquences importantes sur sa commercialisation.

De manière préférée, le milieu de fermentation ne contient pas de glucose et/ou de sulfate d'ammonium ajoutés intentionnellement à la fraction hydrosoluble.

Sans être lié par une quelconque théorie, la déposante a remarqué qu'il était essentiel pour l'invention de réaliser une fermentation aérobie en limitant l'apport d'oxygène, sous peine soit de produire trop d'acide organique dont l'acide lactique, soit de provoquer l'apparition d'une mousse extrêmement importante nécessitant l'apport excessif d'antimousse pour la contrôler. Dans ce dernier cas, l'antimousse représente environ 20% de la matière sèche finale, ce qui est rédhibitoire pour toutes applications alimentaires. Le but n'est donc pas seulement de défructosyler les sucres, mais de le faire en limitant la présence d'acide lactique et/ou d'antimousse, en évitant l'hydrolyse des protéines et en favorisant la synthèse d'agmatine.

Par « micro-organisme », on entend selon l'invention un organisme vivant, invisible à l'œil nu, qui ne peut être observé qu'à l'aide d'un microscope. Les micro-organismes sont représentés par diverses formes de vie parmi lesquelles les bactéries, certains champignons microscopiques, les archéobactéries, les protistes ; des algues vertes microscopiques, des animaux du plancton, les planaires, les amibes... Dans la présente invention, les microorganismes sont des bactéries, préférentiellement du genre *Bacillus.*

Par *« Bacillus »,* on entend selon l'invention le genre de bactéries à gram positif, appartenant à la famille des bacillacées (Bacillaceae), l'ordre des bacillales (Bacillales), la classe des bacilles (Bacillis), le phyllum des firmicutes (Firmicutes). De forme bacilles, les dimensions de ces bactéries sont variables ; elles peuvent aller de (0.5 × 1.2 µm) à (2.5 × 10 µm). Elles sont aérobies ou aéro-anaérobies facultatives, et tirent leur énergie par respiration ou fermentation. Ces bactéries sont capables de produire des endospores leur permettant de résister à des conditions environnementales défavorables. Celles-ci donneront naissance à de nouvelles bactéries en cas de conditions favorables. Les *Bacillus* sont hétérotrophes, saprophytes et ubiquitaires. Elles sont fréquemment retrouvées dans le sol où certaines espèces ont un rôle dans le cycle du carbone et de l'azote. On peut trouver des *Bacillus* dans des denrées alimentaires.

On peut citer comme *Bacillus subtilis* convenant particulièrement à l'invention les *Bacillus subtilis* NRC33a ou bien le *Bacillus subtilis* CCT 7712, qui sont toutes deux des souches décrites dans la littérature.

De manière préférée, la fermentation de la fraction hydrosoluble extraite de légumineuses à l'aide d'un microorganisme du genre *Bacillus* est réalisée à l'aide de la souche de *Bacillus subtilis* telle que déposée le 28 mai 2020 à la CNCM sous le numéro CNCM I-5515. Cette souche a été déposée selon le traité de Budapest auprès de la CNCM de l'Institut Pasteur. La CNCM fait référence à la Collection Nationale de Cultures de Microorganismes de l'Institut Pasteur, située 25 rue du Docteur Roux, F-75724 Paris cedex 15.

De manière plus globale, sans être lié par une quelconque théorie, il est important que la souche possède dans son pool enzymatique une enzyme baptisée levanesucrase. On pourrait ainsi envisager d'autres microorganismes que ceux du genre *Bacillus,* qui restent cependant ceux qui donnent les meilleurs résultats.

Par levanesucrase, on entend au sens de la présente invention l'enzyme sucrose:2,6-beta-D-fructan 6-beta-D-fructosyltransferase (EC 2.4.1.10) qui catalyse la réaction suivante : sucrose + (2,6-beta-D-fructosyl)n -> glucose + (2,6-beta-D-fructosyl)n+1. Cette enzyme appartient à la famille des glycosyltransferases, plus spécialement aux hexosyltransferases.

L'utilisation d'une levanesucrase extraite d'une culture est possible, de manière classique, en solution dans un réacteur, ou bien dans une colonne sous forme d'enzyme immobilisée. Dans ce cas particulier, la production d'agmatine ne sera pas réalisée.

La quatrième étape, optionnelle, du procédé selon l'invention consiste en l'élimination du micro-organisme. Par élimination, on entend préférentiellement l'inactivation du microorganisme, c'est-à-dire une opération visant l'inhibition des processus biochimiques permettant la fermentation et/ou la reproduction. Celle-ci peut être réalisée à l'aide des différentes options bien connues de l'homme du métier, telle que la stérilisation, la pasteurisation ou la filtration membranaire. De manière préférée, l'homme du métier utilisera une pasteurisation, qui permet l'inactivation du microorganisme tout en préservant les molécules labiles présentes. De manière encore plus préférée, l'homme du métier utilisera une centrifugation, qui permet l'élimination du microorganisme tout en ne chauffant pas.

De manière préférée, l'homme du métier, en laissant le microorganisme et/ou ses spores, enrichit la fraction hydrosoluble selon l'invention avec une souche et/ou ses spores à vertu probiotique et/ou postbiotique (probiotique inactivé).

La cinquième et dernière étape, optionnelle, consiste en la stabilisation, préférentiellement au séchage, de la fraction hydrosoluble ainsi obtenue. Toute technique bien connue de l'homme du métier est utilisée. De manière préférée, il utilisera l'atomisation, préférentiellement l'atomisation multiple-effet. D'une manière alternative optionnelle, il concentrera la fraction soluble sous vide à une matière sèche comprise entre 40% et 60%, préférentiellement 50%.

L'invention a enfin pour objet l'utilisation de cette fraction hydrosoluble de légumineuses selon l'invention dans les industries de la nutrition humaine et/ou animale.

L'invention concerne également une souche de *Bacillus subtilis* telle que déposée le 28 mai 2020 à la CNCM sous le numéro I-5515.

L'invention concerne également l'utilisation d'une souche de *Bacillus subtilis* telle que déposée le 28 mai 2020 à la CNCM sous le numéro I-5515 pour la fermentation de glucides, notamment les oligosaccharides, plus particulièrement choisis parmi le raffinose, le stachyose et le verbascose.

L'invention sera mieux comprise à l'aide des exemples non limitatifs suivants.
**Fig 1**

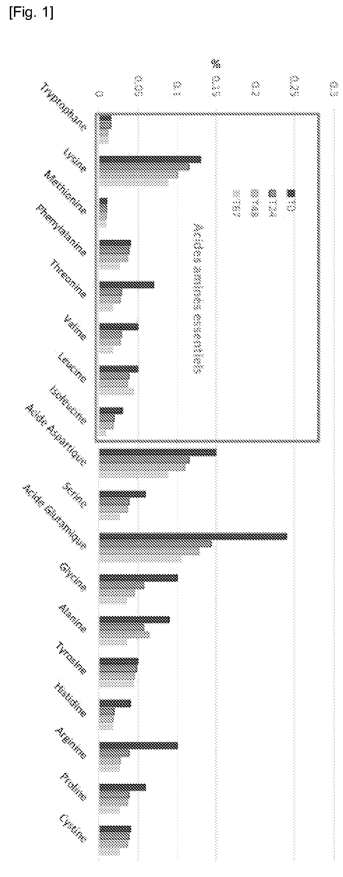

   [Fig. 1] représente la quantification des acides aminés au cours de l'Exemple 3.
**Fig 2**

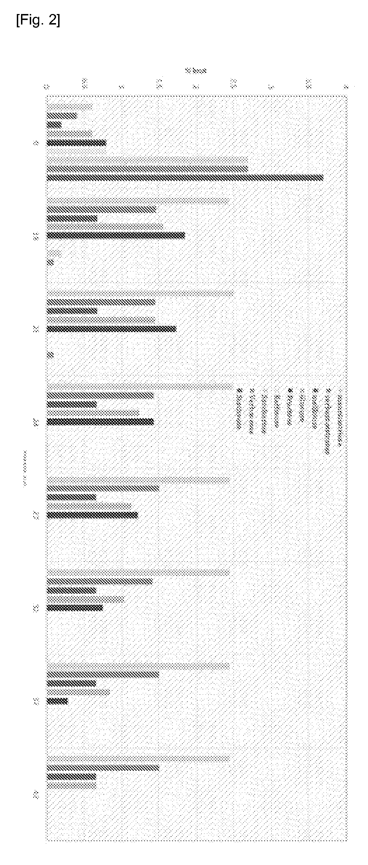

   [Fig. 2] représente la quantification des différents sucres au cours de l'Exemple 4.
**Fig 3**

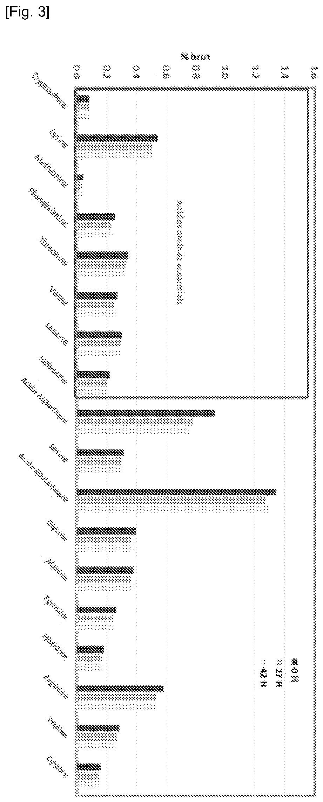

   [Fig. 3] représente la quantification des acides aminés au cours de l'Exemple 4.

### Exemples

### Exemple 1 : Production d'une fraction hydrosoluble comme matière première

On utilise des graines de pois pour cet exemple. Après décorticage des fibres externes sur broyeur à marteaux, on broie les cotylédons obtenus afin d'obtenir une farine. 1044 kg de suspension de farine à 25 % en poids de matière sèche (soit donc 261 kg de farine sèche) est alors introduite avec 500 kg d'eau dans une batterie d'hydrocyclones adaptée d'une unité industrielle féculière de traitement de la pomme de terre. Cette séparation conduit à l'obtention d'une phase légère constituée du mélange protéines, fibres internes et solubles. La phase lourde, renfermant l'amidon est mise de côté.

La phase légère en sortie d'hydrocyclones renferme quant à elle en mélange (142 kg en poids sec au total) : les fibres (environ 14,8 % en poids, soit 21 kg sec), les protéines (environ 42,8 % en poids, soit 60,8 kg sec) et de solubles (environ 42,4 % en poids, soit 60,2 kg sec). On l'amène ensuite à une teneur en matière sèche de 11,4 %. On procède à la séparation des fibres sur décanteurs centrifuges de type WESPHALIA employés dans une unité industrielle féculière de traitement de la pomme de terre. La phase légère en sortie de décanteur centrifuge renferme un mélange de protéines et de solubles, tandis que la phase lourde renferme les fibres de pois. La phase lourde renferme 105 kg de fibres à 20 % en poids de matière sèche. On constate que la quasi-totalité des fibres est bien retrouvée dans cette fraction. Cette fraction sera dénommée ci-après « Fibres internes du pois » et correspond à la fraction pulpes.

Quant à la fraction légère, elle renferme 1142 kg d'un mélange en solution de solubles et de protéines. On procède à la coagulation des protéines à leur point isoélectrique par ajustement de la phase légère de sortie de décanteur centrifuge à un pH de 4,6 et chauffage à 70°C de cette solution pendant 20 min. Après précipitation des protéines, on élimine le sédiment renfermant 56 kg de protéines (86 % de N 6,25 sur sec). La fraction liquide qui sera dénommée « Fraction hydrosoluble» est concentrée par évaporation sous vide à environ 30% en poids de MS.

La teneur en GOS non-defructosylés est de 22,9 g/100g de MS.

La quantité de protéines est de 30,1 g/100g de MS.

On calcule le degré d'hydrolyse (ou DH) de ces protéines selon la méthode OPA dont le protocole est décrit dans la présente demande. Ce DH est égal à 11.

### Exemple 2 : Fermentation de la fraction hydrosoluble obtenue dans l'exemple 1 avec un procédé hors invention (aération par le dôme du fermenteur):

Une souche *Bacillus subtilis,* telle que déposée à la CNCM sous le numéro CNCM I-5515, est utilisée pour réaliser la fermentation de la fraction hydrosoluble.

Un cryotube de 5ml contenant 10⁸ UFC/ml est utilisé pour ensemencer un erlen de 2L à baffles contenant 500 ml de milieu LB (Tryptone (Bacto Trypton)10 g/l, extrait de levures (BactoYest Extract) 5 g/L et chlorure de sodium (NaCl) 10 g/L, Stérilisation 20min à 120°C). Cet erlen est mis à incuber à 37°C sous une agitation de 150 RPM durant 4,5 heures.

La production est réalisée dans un fermenteur d'un volume de 15L après inoculation (7% de préculture), les paramètres de fermentation sont les suivants : l'agitation est fixée à 300 RPM (rotations par minutes); débit d'air=4L/mn dans le ciel du fermenteur. Le pH est régulé à 7 par ajout de soude à 25%. La température est régulée à 37°C.

La pO2 n'a pas été suivie du fait de l'aération en surface du milieu : le principe d'aération par le dôme du fermenteur entraine par conséquence que la pO2 du milieu de fermentation est nulle. Une première phase de croissance se distingue en observant le CPR (CPR signifie la quantité de CO2 émis par la souche) qui augmente dès le début de la fermentation jusqu'à 15H pour diminuer ensuite à 30H. Cette émission de CO2 démontre que le métabolisme de la souche lors de cette fermentation était de type fermentaire et que donc celle-ci n'utilisait pas ou très peu l'oxygène de l'air. Puis une deuxième phase de croissance qui démarre à 30H jusqu'à la fin de fermentation, à 40h. Ce phénomène de diauxie est de plus observé par le profil de l'ajout de base.

Le mout issu du fermenteur de production est atomisé. On commence par centrifuger (30000 G, 20mn) pour récupérer le surnageant, puis ce dernier est évaporé pour atteindre environ 10% de matière sèche et assurer une atomisation correcte. Les paramètres d'atomisation sont les suivants : T°C en entrée 190°C, T°C en sortie 110°C.

On suit l'évolution des différents sucres à l'aide d'une chromatographie sur couche mince (CCM) et par HPLC (HPAEC-PAD). à T = 0H on constate la présence de raffinose, de stachyose et de verbascose, qui forment les GOS (galactooligosaccharides). Des points de suivi ont été effectués à 19H, 24H et 42H de fermentation. On constate que les GOS sont progressivement et entièrement défructosylés. Le raffinose se transforme en mélibiose, le stachyose en manninotriose et le verbascose en verbascotétraose. Le saccharose étant entièrement consommé.

Le profil en acide aminés est plutôt bien conservé, à l'exception de l'arginine. Ce profil en acide aminé est obtenu par hydrolyse des protéines et analyse HPLC classique bien connue de l'homme du métier.

Une analyse des acides organiques produits par HPLC montre une teneur élevée en acide organiques dont 12% sur sec en acide lactique, ce qui est trop élevé pour certaines applications, sans envisager une purification additionnelle.

On peut donc conclure que la défructosylation se déroule bien, sans moussage important, mais la production d'acide lactique à hauteur de 12% est un handicap supplémentaire.

La teneur en GOS défructosylés est de 16,5 %.

La quantité de protéines est de 29,8 %.

### Exemple 3 : Fermentation de la fraction hydrosoluble selon l'invention avec un procédé hors invention (aération classique dans le milieu de fermentation, avec contrôle de la p02)

Une souche *Bacillus subtilis,* telle que déposée à la CNCM sous le numéro CNCM I-5515, est utilisée pour réaliser la fermentation de la fraction hydrosoluble.

Un cryotube de 5ml contenant 10⁸ UFC/ml est utilisé pour ensemencer un erlen de 2L à baffles contenant 500 ml de milieu LB (Tryptone (Bacto Trypton)10 g/l, extrait de levures (BactoYest Extract) 5 g/L et chlorure de sodium (NaCl) 10 g/L, Stérilisation 20min à 120°C). Cet erlen est mis à incuber à 37°C sous une agitation de 150 RPM durant 4,5 heures.

La production est réalisée dans un fermenteur d'un volume de 15L après inoculation (10% de préculture), les paramètres de fermentation sont les suivants : La PO2 est régulée à 30% en cascade sur l'agitation (c'est-à-dire régulation de la p02 à l'aide de l'agitation), le minimum d'agitation est de 400 RPM; débit d'air de 1VVM, envoyé directement dans le milieu liquide. Le pH est régulé à 7 par ajout de soude à 25%. La température est régulée à 37°C.

Aucun temps d'adaptation n'est observé. La pO2 chute très rapidement de 100% à 30% en 4H. Dès 2H de production, il y a formation de mousse, l'apport massif d'anti-mousse est réalisé dans le milieu via une pompe idoine. Sans cet apport d'antimousse, le fermenteur se vide entièrement (réalisé dans des essais préliminaires). Cet ajout, que l'on quantifie par pesée, est constant jusqu'à 13 heures de fermentation, temps auquel la pO2 remonte rapidement. Puis cette dernière stagne à 80% entre 15H et 20H de fermentation, qui pourrait témoigner d'une phase de diauxie. Une pO2 supérieure à 30% a pu être maintenue sans augmentation de l'agitation. L'analyse des gaz confirme les observations faites ci-dessus sur la diauxie.

Le mout issu du fermenteur de production est atomisé. On commence par centrifuger (30000 G, 20mn) pour récupérer le surnageant, puis ce dernier est évaporé pour atteindre environ 10% de matière sèche et assurer une atomisation correcte. Les paramètres d'atomisation sont les suivants : T°c en entrée 190°C, T°c en sortie 110°C.

On suit l'évolution des différents sucres à l'aide d'une chromatographie sur couche mince (CCM) et par HPLC (HPAEC-PAD) .à T = 0H on constate la présence de raffinose, de stachyose et de verbascose, qui forment les GOS. Des points ont été effectués à 19H, 24H et 42H de fermentation. On constate que dès 19H, ces GOS sont progressivement et entièrement défructosylés. Le raffinose est transformé en mélibiose, le stachyose en manninotriose et le verbascose en verbascotétraose. Le saccharose étant entièrement consommé.

Comme le montre la Figure 1, le profil en acides aminés est particulièrement modifié, avec perte d'une quantité importante de ceux-ci. Ce profil en acide aminé est obtenu par hydrolyse des protéines et analyse HPLC classique bien connue de l'homme du métier.

La teneur en GOS est de 17,5 %

La quantité de protéines est de 26,1 %.

On calcule également le degré d'hydrolyse (ou DH) selon la méthode OPA dont le protocole est décrit dans la présente demande. Ce DH est égal à 20,5.

En réalisant un bilan massique des substrats utilisés et des quantités d'antimousse envoyé dans le fermenteur, on estime environ à 20% la quantité finale d'antimousse dans le fermenteur. Cette quantité est bien trop importante pour envisager une valorisation directe sans purification de cette fraction.

La perte d'acides aminés, l'augmentation du DH et la quantité résiduelle d'antimousse disqualifient ce mode de production, en altérant la qualité nutritionnelle, malgré la défructosylation.

### Exemple 4 : Fermentation de la fraction hydrosoluble avec un procédé selon invention par aération dans le milieu de fermentation, avec un débit d'aération controlé et une pO2 nulle :

Une souche *Bacillus subtilis,* telle que déposée à la CNCM sous le numéro CNCM I-5515, est utilisée pour réaliser la fermentation de la fraction hydrosoluble.

Un cryotube de 5ml contenant 10⁸ UFC/ml est utilisé pour ensemencer un erlen de 2L à baffles contenant 500 ml de milieu LB (Tryptone (Bacto Trypton)10 g/l, extrait de levures (BactoYest Extract) 5 g/L et chlorure de sodium (NaCl) 10 g/L, Stérilisation 20min à 120°C). Cet erlen est mis à incuber à 37°C sous une agitation de 150 RPM durant 4,5 heures.

La production est réalisée dans un fermenteur d'un volume de 15L après inoculation (10% de préculture), les paramètres de fermentation sont les suivants : Débit d'air de 0,25 VVM, sans contrôle de la p02 directement dans le milieu liquide. L'agitation est fixée à 300 RPM. Le pH est rectifié à 6 avec soude et acide chlorhydrique mais n'est pas régulé ensuite.

Aucun temps d'adaptation n'est observé. La pO2 chute très rapidement pour rester nulle jusqu'à la fin de fermentation. Le pH rectifié à 6 reste invarié. La fermentation est réalisée pendant 42h, avec différents prélèvements en cours de fermentation.

Le mout issu du fermenteur de production est atomisé. On commence par centrifuger (30000 G, 20mn) pour récupérer le surnageant, puis ce dernier est évaporé pour atteindre environ 10% de matière sèche et assurer une atomisation correcte. Les paramètres d'atomisation sont les suivants : T°c en entrée 190°C, T°c en sortie 110°C.

On suit l'évolution des différents sucres à l'aide d'une chromatographie sur couche mince (CCM) et par HPLC (HPAEC-PAD) à T = 0H on constate la présence de raffinose, de stachyose et de verbascose, qui forment les GOS. Des points ont été effectués à 19H, 24H et 42H de fermentation. On constate que dès 19H, ces GOS sont entièrement défructosylés. Le raffinose est transformé en mélibiose, le stachyose en manninotriose et le verbascose en verbascotétraose. Le saccharose étant entièrement consommé.

La teneur en acide lactique analysée par HPLC est dosée à 1% en poids sec d'acide lactique par poids total. Dans un essai alternatif similaire en tout point à celui-ci mais différent uniquement en ce que le pH de la fermentation ait été rectifié à 7 au lieu de 6, la teneur en acide lactique est dosée à 5%.

Le profil d'acides aminés, également obtenu par HPLC, est bien conservé.

La teneur en GOS est de 18,3%.

La quantité de protéines est de 30,5%.

On calcule le degré d'hydrolyse (ou DH) selon la méthode OPA dont le protocole est décrit dans la présente demande. Ce DH est égal à 11,5.

### Exemple 5 : Défructosylation de l'art antérieur avec une invertase selon la demande de brevet WO2010/109093

La fraction hydrosoluble de pois est ajustée à 15% en poids de matière sèche et filtrée au moyen d'une membrane d'ultrafiltration, avec un seuil de coupe fixé à 5.000 Da, en vue de la clarifier et d'en éliminer les protéines. Cette étape est suivie d'une concentration du perméat par osmose inverse, pour le ramener à 20% en poids de matière sèche.

Parallèlement, on prépare 100 ml d'une solution d'invertase à 1 mg/ml, qui est ensuite également lavée par centrifugation pendant 30 minutes. Le culot est repris par 50 ml d'eau. On mélange alors 980 ml de la fraction de pois avec 50 ml de la solution enzymatique, dans un réacteur à double paroi et agitateur placé au bain-marie à 50°C. L'hydrolyse est contrôlée par dosage des sucres réducteurs à l'aide d'une solution alcaline aqueuse d'acide 3,5-dinitrosalicylique (DNS), à différents intervalles de temps. Après au moins 12h d'hydrolyse, l'enzyme est neutralisée, puis le produit obtenu est centrifugé puis filtré pour obtenir une solution limpide qui est ensuite concentrée par évaporation rotative sous vide à 70°C, jusqu'à l'obtention d'un jus limpide.

Une analyse du fructose total dans le produit obtenu montre une teneur en fructose très importante, environ 10%.

## Revendications

1. Fraction hydrosoluble extraite de légumineuses comportant entre 10% et 30% d'oligosaccharides défructosylés, préférentiellement entre 10% et 25%, préférentiellement entre 15% et 25%, encore plus préférentiellement entre 20% et 22%, et entre 20% et 40% de protéines, préférentiellement entre 25% et 35%, encore plus préférentiellement 30%, les pourcentages étant exprimés en poids sec par rapport au poids total de matière sèche.

2. Fraction hydrosoluble extraite de légumineuses selon la revendication 1 **caractérisée en ce que** les légumineuses sont sélectionnées parmi la liste constituée du pois et de la féverole, encore plus préférentiellement du pois.

3. Fraction hydrosoluble extraite de légumineuses selon l'une quelconque des revendications 1 ou 2 **caractérisée en ce qu'**elle comporte entre entre 10% et 30% d'oligosaccharides défructosylés, préférentiellement entre 10% et 25%, préférentiellement entre 15% et 25%, encore plus préférentiellement entre 20% et 22% sélectionnés dans la liste constituée par le mélibiose, le manninotriose et le manninotétraose.

4. Fraction hydrosoluble extraite de légumineuses selon l'une quelconque des revendications 1 à 3 **caractérisée en ce qu'**elle contient moins de 2 %, de manière préférée entre 0,25% et 1%, encore plus préférentiellement entre 0,5% et 0,75% en poids de fructose, les pourcentages étant exprimés en poids par rapport au poids total de matière sèche.

5. Fraction hydrosoluble extraite de légumineuses selon l'une quelconque des revendications 1 à 4 **caractérisée en ce qu'**elle contient moins de 2 %, de manière préférée entre 0,25% et 1%, encore plus préférentiellement entre 0,5% et 0,75% en poids de lactate, les pourcentages étant exprimés en poids par rapport au poids total de matière sèche.

6. Fraction hydrosoluble extraite de légumineuses selon l'une quelconque des revendications 1 à 5 **caractérisée en ce qu'**elle comporte entre 20% et 40% de protéines, préférentiellement entre 25% et 35%, encore plus préférentiellement 30%, caractérisées comme des albumines.

7. Fraction hydrosoluble extraite de légumineuses selon l'une quelconque des revendications 1 à 6 **caractérisée en ce qu'**elle possède des protéines, préférentiellement des albumines, dont le degré d'hydrolyse, ou DH, est inférieur à 20, préférentiellement inférieur à 18, encore plus préférentiellement inférieur à 15.

8. Fraction hydrosoluble extraite de légumineuses selon l'une quelconque des revendications 1 à 7 **caractérisée en ce qu'**elle comporte également de l'agmatine dans une concentration comprise entre 10 ppm et 100 ppm exprimé en poids sec d'agmatine sur poids sec de produit final, préférentiellement entre 10 ppm et 40 ppm sur sec, préférentiellement entre 15 ppm et 35 ppm, encore plus préférentiellement entre 20 ppm et 30 ppm.

9. Procédé d'obtention d'une fraction hydrosoluble extraite de légumineuses comportant entre 10% et 30% d'oligosaccharides défructosylés et entre 20% et 40% de protéines, ledit procédé comportant les étapes suivantes :
1- Obtention d'une fraction hydrosoluble de légumineuses,
2- Optionnellement, dessalement de la fraction hydrosoluble,
3- Fermentation de la fraction hydrosoluble extraite de légumineuses à l'aide d'un microorganisme du genre *Bacillus,* préférentiellement *Bacillus subtilis,* ladite fermentation étant une fermentation en aérobie avec une pO2 nulle, tout en apportant de l'oxygène au milieu de fermentation directement dans le liquide,
4- Optionnellement, élimination du micro-organisme,
5- Optionnellement, stabilisation bactériologique de la fraction hydrosoluble ainsi obtenue.

10. Procédé selon la revendication 9 **caractérisé en ce que** la fraction hydrosoluble de l'étape 1 est obtenue par le procédé suivant :
i) Mise en œuvre de graines de légumineuses, avec un pré-traitement optionnel ;
ii) Séparation par voie humide des constituants des graines de légumineuses en 4 fractions : une fraction amidon, une fraction pulpes, une fraction protéines de type globulines et une fraction hydrosoluble soluble résiduelle

11. Procédé selon l'une quelconque des revendications 9 à 10 **caractérisé en ce que** le milieu de fermentation de l'étape 3 ne comprend que la fraction hydrosoluble de légumineuse.

12. Procédé selon l'une quelconque des revendications 9 à 11, **caractérisé en ce que** l'étape de fermentation de l'étape 3 est réalisée par introduction d'un débit d'air compris entre 0,03 VVM et 0,5 VVM, préférentiellement entre 0,1 VVM et 0,4 VVM, encore plus préférentiellement entre 0,2 VVM et 0,3 VVM, 0,25 VVM étant la valeur ciblée préférentiellement.

13. Procédé selon l'une quelconque des revendications 9 à 12 **caractérisé en ce que** le pH de la fermentation est rectifié voire régulé entre 5,5 et 6,5 ; préférentiellement 6.

14. Procédé selon l'une quelconque des revendications 9 à 13, **caractérisé en ce que** l'étape de fermentation est réalisée à l'aide de la souche de *Bacillus subtilis* telle que déposée le 28 mai 2020 à la CNCM sous le numéro CNCM I-5515.

15. Utilisation de la fraction hydrosoluble extraite de légumineuses selon l'une quelconque des revendications 1 à 8 ou obtenue selon le procédé de l'une quelconque des revendications 9 à 14 dans les industries de la nutrition humaine et/ou animale.

16. Souche de Bacillus subtilis telle que déposée le 28 mai 2020 à la CNCM sous le numéro I-5515.

## Patentansprüche

1. Aus Leguminosen extrahierte wasserlösliche Fraktion, umfassend zwischen 10 % und 30 %, vorzugsweise zwischen 10 % und 25 %, vorzugsweise zwischen 15 % und 25 %, noch mehr bevorzugt zwischen 20 % und 22 %, defructosylierte Oligosaccharide und zwischen 20 % und 40 %, vorzugsweise zwischen 25 % und 35 %, noch mehr bevorzugt 30 %, Proteine; wobei die Prozentsätze in Trockengewicht bezogen auf das Gesamtgewicht der Trockensubstanz ausgedrückt sind.

2. Aus Leguminosen extrahierte wasserlösliche Fraktion nach Anspruch 1, **dadurch gekennzeichnet, dass** die Leguminosen ausgewählt sind aus der Liste bestehend aus Erbse und Ackerbohne, noch mehr bevorzugt aus Erbse.

3. Aus Leguminosen extrahierte wasserlösliche Fraktion nach einem der Ansprüche 1 oder 2, **dadurch gekennzeichnet, dass** sie zwischen 10 % und 30 %, vorzugsweise zwischen 10 % und 25 %, vorzugsweise zwischen 15 % und 25 %, noch mehr bevorzugt zwischen 20 % und 22 %, defructosylierte Oligosaccharide ausgewählt aus der Liste bestehend aus Melibiose, Manninotriose und Manninotetraose umfasst.

4. Aus Leguminosen extrahierte wasserlösliche Fraktion nach einem der Ansprüche 1 bis 3, **dadurch gekennzeichnet, dass** sie weniger als 2 Gew.-%, vorzugsweise zwischen 0,25 Gew.-% und 1 Gew.-%, noch mehr bevorzugt zwischen 0,5 Gew.-% und 0,75 Gew.-%, Fructose enthält, wobei die Prozentsätze als Gewicht bezogen auf das Gesamtgewicht der Trockenmasse ausgedrückt sind.

5. Aus Leguminosen extrahierte wasserlösliche Fraktion nach einem der Ansprüche 1 bis 4, **dadurch gekennzeichnet, dass** sie weniger als 2 Gew.-%, vorzugsweise zwischen 0,25 Gew.-% und 1 Gew.-%, noch mehr bevorzugt zwischen 0,5 Gew.-% und 0,75 Gew.-%, Laktat enthält, wobei die Prozentsätze als Gewicht bezogen auf das Gesamtgewicht der Trockenmasse ausgedrückt sind.

6. Aus Leguminosen extrahierte wasserlösliche Fraktion nach einem der Ansprüche 1 bis 5, **dadurch gekennzeichnet, dass** sie zwischen 20 % und 40 %, vorzugsweise zwischen 25 % und 35 %, noch mehr bevorzugt 30 %, Proteine umfasst, die zur Gruppe der Albumine gehören.

7. Aus Leguminosen extrahierte wasserlösliche Fraktion nach einem der Ansprüche 1 bis 6, **dadurch gekennzeichnet, dass** sie Proteine, vorzugsweise Albumine, aufweist, deren Hydrolysegrad (bzw. DH) kleiner als 20, vorzugsweise kleiner als 18, noch mehr bevorzugt kleiner als 15, ist.

8. Aus Leguminosen extrahierte wasserlösliche Fraktion nach einem der Ansprüche 1 bis 7, **dadurch gekennzeichnet, dass** sie ferner Agmatin in einer Konzentration zwischen 10 ppm und 100 ppm, ausgedrückt in Trockengewicht von Agmatin bezogen auf das Trockengewicht des Endprodukts, vorzugsweise zwischen 10 ppm und 40 ppm bezogen auf das Trockengewicht, vorzugsweise zwischen 15 ppm und 35 ppm, noch mehr bevorzugt zwischen 20 ppm und 30 ppm, umfasst.

9. Verfahren für die Gewinnung einer aus Leguminosen extrahierten wasserlöslichen Fraktion, umfassend zwischen 10 % und 30 % defructosylierte Oligosaccharide und zwischen 20 % und 40 % Proteine, das Verfahren die folgenden Schritte umfassend:
1- Gewinnen einer wasserlöslichen Fraktion aus Leguminosen,
2- Optional, Entsalzen der wasserlöslichen Fraktion,
3- Fermentieren der aus Leguminosen extrahierten wasserlöslichen Fraktion mittels eines Mikroorganismus der Gattung *Bacillus,* vorzugsweise *Bacillus subtilis,* wobei die Fermentation eine aerobe Fermentation mit einem pO2 von null ist, während dem Fermentationsmedium Sauerstoff direkt in die Flüssigkeit zugeführt wird,
4- Optional, Entfernen des Mikroorganismus,
5- Optional, bakteriologisches Stabilisieren der auf diese Weise erhaltenen wasserlöslichen Fraktion.

10. Verfahren nach Anspruch 9, **dadurch gekennzeichnet, dass** die wasserlösliche Fraktion aus Schritt 1 durch das folgende Verfahren erhalten wird:
i) Bereitstellen von Leguminosensamen mit einer optionalen Vorbehandlung;
ii) Nasssortieren der Bestandteile von Leguminosensamen in 4 Fraktionen: einer Stärkefraktion, einer Pulpefraktion, einer Proteinfraktion vom Typ Globuline und einer wasserlöslichen löslichen Restfraktion

11. Verfahren nach einem der Ansprüche 9 bis 10,
**dadurch gekennzeichnet, dass** das Fermentationsmedium aus Schritt 3 nur die wasserlösliche Leguminosen-Fraktion umfasst.

12. Verfahren nach einem der Ansprüche 9 bis 11,
**dadurch gekennzeichnet, dass** der Schritt der Fermentation aus Schritt 3 durch Einleiten eines Luftdurchsatzes zwischen 0,03 VVM und 0,5 VVM, vorzugsweise zwischen 0,1 VVM und 0,4 VVM, noch mehr bevorzugt zwischen 0,2 VVM und 0,3 VVM durchgeführt wird, wobei 0,25 VVM der vorzugsweise angestrebte Wert ist.

13. Verfahren nach einem der Ansprüche 9 bis 12,
**dadurch gekennzeichnet, dass** der pH-Wert der Fermentation zwischen 5,5 und 6,5, vorzugsweise 6, eingestellt oder sogar geregelt wird.

14. Verfahren nach einem der Ansprüche 9 bis 13,
**dadurch gekennzeichnet, dass** der Schritt der Fermentation mittels des Stammes von *Bacillus subtilis*, wie am 28. Mai 2020 bei der CNCM unter der Nummer CNCM I-5515 hinterlegt, durchgeführt wird.

15. Verwendung der aus Leguminosen extrahierten wasserlöslichen Fraktion nach einem der Ansprüche 1 bis 8 oder, die gemäß dem Verfahren nach einem der Ansprüche 9 bis 14 erhalten wurde, in der Industrie für menschliche und/oder tierische Ernährung.

16. Stamm von Bacillus subtilis, wie er am 28. Mai 2020 bei der CNCM unter der Nummer I-5515 hinterlegt wurde.

## Claims

1. A hydrosoluble fraction extracted from leguminous plants comprising between 10% and 30% of defructosylated oligosaccharides, preferentially between 10% and 25%, preferentially between 15% and 25%, even more preferentially between 20% and 22%, and between 20% and 40% of proteins, preferentially between 25% and 35%, even more preferentially 30%, the percentages being expressed as dry weight relative to the total weight of solids.

2. The hydrosoluble fraction extracted from leguminous plants according to claim 1, **characterized in that** the leguminous plants are selected from the list consisting of pea and faba bean, even more preferentially of pea.

3. The hydrosoluble fraction extracted from leguminous plants according to either one of claims 1 or 2, **characterized in that** it comprises between 10% and 30% of defructosylated oligosaccharides, preferentially between 10% and 25%, preferentially between 15% and 25%, even more preferentially between 20% and 22% selected from the list consisting of melibiose, manninotriose, and manninotetraose.

4. The hydrosoluble fraction extracted from leguminous plants according to any one of claims 1 to 3, **characterized in that** it contains less than 2%, preferably between 0.25% and 1%, even more preferentially between 0.5% and 0.75% by weight of fructose, the percentages being expressed as weight relative to the total weight of solids.

5. The hydrosoluble fraction extracted from leguminous plants according to any one of claims 1 to 4, **characterized in that** it contains less than 2%, preferably between 0.25% and 1%, even more preferentially between 0.5% and 0.75% by weight of lactate, the percentages being expressed as weight relative to the total weight of solids.

6. The hydrosoluble fraction extracted from leguminous plants according to any one of claims 1 to 5, **characterized in that** it comprises between 20% and 40% of proteins, preferentially between 25% and 35%, even more preferentially 30%, characterized as albumins.

7. The hydrosoluble fraction extracted from leguminous plants according to any one of claims 1 to 6, **characterized in that** it has proteins, preferentially albumins, the degree of hydrolysis, or DH, of which is less than 20, preferentially less than 18, even more preferentially less than 15.

8. The hydrosoluble fraction extracted from leguminous plants according to any one of claims 1 to 7, **characterized in that** it also comprises agmatine at a concentration of between 10 ppm and 100 ppm expressed as dry weight of agmatine to dry weight of final product, preferentially between 10 ppm and 40 ppm on dry basis, preferentially between 15 ppm and 35 ppm, even more preferentially between 20 ppm and 30 ppm.

9. A process for obtaining a hydrosoluble fraction extracted from leguminous plants comprising between 10% and 30% of defructosylated oligosaccharides and between 20% and 40% of proteins, said process comprising the following steps:
1- Obtaining a hydrosoluble fraction from leguminous plants,
2- Optionally, desalinating the hydrosoluble fraction,
3- Fermenting the hydrosoluble fraction extracted from leguminous plants using a microorganism of the genus *Bacillus,* preferably *Bacillus subtilis,* said fermentation being aerobic fermentation at zero pO2, while supplying oxygen to the fermentation medium directly into the liquid,
4- Optionally, eliminating the microorganism,
5- Optionally, bacteriologically stabilizing the hydrosoluble fraction thus obtained.

10. The process according to claim 9, **characterized in that** the hydrosoluble fraction of step 1 is obtained by the following process:
i) Implementing leguminous plant seeds, with an optional pre-treatment;
ii) Wet-separating the constituents of the leguminous plant seeds into 4 fractions: a starch fraction, a pulp fraction, a protein fraction of globulin type, and a residual hydrosoluble fraction

11. The process according to either one of claims 9 to 10,
**characterized in that** the fermentation medium of step 3 comprises only the hydrosoluble fraction of leguminous plants.

12. The process according to any one of claims 9 to 11, **characterized in that** the fermentation step of step 3 is carried out by introducing an air flow of between 0.03 VVM and 0.5 VVM, preferentially between 0.1 VVM and 0.4 VVM, even more preferentially between 0.2 VVM and 0.3 VVM, 0.25 VVM being the preferentially targeted value.

13. The process according to any one of claims 9 to 12, **characterized in that** the pH of the fermentation is rectified or adjusted between 5.5 and 6.5; preferentially 6.

14. The process according to any one of claims 9 to 13, **characterized in that** the fermentation step is carried out using the strain of *Bacillus subtilis* as deposited with the CNCM on May 28, 2020 under CNCM number I-5515.

15. The use of the hydrosoluble fraction extracted from leguminous plants according to any one of claims 1 to 8 or obtained by the process of any one of claims 9 to 14 in the human and/or animal nutrition industries.

16. A strain of Bacillus subtilis as deposited with the CNCM on May 28, 2020 under the number I-5515.
